# EUROPEAN PATENT APPLICATION

(11) **EP 4 389 132 A1**
(43) Date of publication of application: **26.06.2024**
(21) Application number: 22858544.4
(22) Date of filing: 19.08.2022
(51) Int. Cl.: A61K 31/702, A23K 20/163, A61K 31/7004, A61P 31/12, A61P 31/14, A61P 31/18, D06M 13/148

(54) **VIRUS-INFECTION SUPPRESSION AGENT**

(30) Priority: 20.08.2021 JP 2021135060
(71) Applicant: Resonac Corporation, Tokyo 105-7325 (JP)
(72) Inventor: NAKAGAMI Yuko, Tokyo 105-7325 (JP)
(74) Representative: Strehl Schübel-Hopf & Partner
(86) International application number: PCT/JP2022/031363
(87) International publication number: WO 2023/022227

(57) **Abstract**

A viral infection suppressant is provided, containing, as an active component, an inositol derivative in which a sugar is bound to inositol. In addition, a method of use is provided, including applying the viral infection suppressant to a surface of an article. In addition, a method of use is provided, including spraying the viral infection suppressant on a body surface of an animal. In addition, a method of use is provided, including spraying or blending the antiviral infection suppressant into a fiber. In addition, a composition for suppressing viral infection is provided, including: the viral infection suppressant; and a medicinally acceptable carrier.

## Description

### [Technical Field]

The present invention relates to a viral infection suppressant. In addition, the present invention also relates to a method of using a viral infection suppressant and a composition for preventing viral infection.
Priority is claimed on Japanese Patent Application No. 2021-135060, filed August 20, 2021, the content of which is incorporated herein by reference.

### [Background Art]

SARS-related coronavirus (hereinafter referred to as "SARS virus") is a coronavirus that causes severe acute respiratory syndrome (SARS). The virus (SARS-CoV-2) that causes COVID-19, which has become a worldwide infection problem as of 2021, is a type of SARS virus.

The SARS virus infects a target cell by fusing its own cell membrane with the cell membrane of the target cell. The SARS virus has a spike protein (hereinafter referred to as "S protein") classified as a class I membrane fusion-promoting protein on its membrane surface. This S protein is known to play a major role in membrane fusion to target cells.

To suppress infection by viruses including the SARS virus, it is effective to inhibit the binding of viruses to target cells. For this purpose, peptides that specifically recognize binding portions of viruses to target cells (Patent Documents 1 and 2) and algae-derived lectins (Patent Document 3) have been reported as compounds that have the effect of suppressing viral infection. It is expected that specific sequences of these peptides and lectins will inhibit the binding recognition of target viruses with respect to target cells (Patent Documents 1 to 3).

### [Citation List]

### [Patent Documents]

[Patent Document 1]
   Japanese Unexamined Patent Application, First Publication No. 2007-326781
[Patent Document 2]
   Japanese Unexamined Patent Application, First Publication No. 2007-129942
[Patent Document 3]
   Japanese Unexamined Patent Application, First Publication No. 2021-13375

### [Summary of Invention]

### [Technical Problem]

Suppressing infection with SARS viruses, especially SARS-CoV-2, is an urgent global issue. For this reason, viral infection suppressants which have an effect of suppressing infection with SARS-CoV-2 and can be easily used for various daily applications have come to be required.

Therefore, an object of the present invention is to provide a viral infection suppressant, a method of using the same, and a composition for preventing viral infection which has an effect of suppressing infection with SARS viruses, especially SARS-CoV-2, and can be easily used for various daily applications.

### [Solution to Problem]

The present invention has the following aspects.
[1] A viral infection suppressant containing, as an active component, an inositol derivative in which a sugar is bound to inositol.
[2] The viral infection suppressant according to [1], in which the sugar is glucose or an oligosaccharide containing glucose as a constituent unit.
[3] The viral infection suppressant according to [1] or [2], in which the inositol is myo-inositol.
[4] The viral infection suppressant according to any one of [1] to [3], in which the virus is at least one virus selected from the group consisting of the family *Orthomyxoviridae,* the family *Paramyxoviridae*, the family *Retroviridae*, the family *Filoviridae*, and the family *Coronaviridae.*
[5] The viral infection suppressant according to [4], in which the virus is SARS-related coronavirus.
[6] A method of using a viral infection suppressant, the method including: applying the viral infection suppressant according to any one of [1] to [5] to a surface of an article.
[7] A method of using a viral infection suppressant, the method including: spraying the viral infection suppressant according to according to any one of [1] to [5] on a body surface of an animal.
[8] A method of using a viral infection suppressant, the method including: spraying or blending the viral infection suppressant according to any one of [1] to [5] into a fiber.
[9] A composition for suppressing viral infection, including: the viral infection suppressant according to any one of [1] to [5]; and a pharmaceutically acceptable carrier.
[10] The composition for suppressing viral infection according to [9], in which a total content of the inositol derivative is 0.1 to 5.0 mass%.

### [Advantageous Effects of Invention]

According to the present invention, it is possible to provide a viral infection suppressant that can be easily used for various daily applications. In addition, it is possible to provide a method of using a viral infection suppressant and a composition for preventing viral infection containing the viral infection suppressant.

### [Description of Embodiments]

Hereinafter, embodiments of the present invention will be described in detail. However, the present invention is not limited to the embodiments shown below.

### (Viral infection suppressant)

A viral infection suppressant of the present embodiment contains an inositol derivative in which a sugar is bound to inositol (hereinafter also simply referred to as "inositol derivative"). The inositol derivative has an effect of suppressing binding of viruses to target cells. The inositol derivative has an effect of suppressing, for example, S protein of a SARS virus containing SARS-CoV-2 from binding to ACE2 protein on the surface of human cells. For this reason, when an inositol derivative is used as a viral infection suppressant, an effect of suppressing viruses, especially SARS viruses, from infecting human cells can be expected.

### <lnositol derivative>

The inositol derivative in the viral infection suppressant of the present embodiment is a compound composed of inositol and a sugar, and specifically, is a compound in which a sugar is bound to at least one hydroxyl group of inositol.

### • Inositol

Inositol constituting the inositol derivative is a cyclic hexahydric alcohol represented by C₆H₆(OH)₆. Inositol has nine stereoisomers: cis-inositol, epi-inositol, allo-inositol, myo-inositol, muco-inositol, neo-inositol, chiro-inositol (there are D- and L-forms), and scyllo-inositol.

Among the stereoisomers described above, myo-inositol, which has physiological activity, is preferable as inositol constituting the inositol derivative. A structural formula of myo-inositol is shown below.

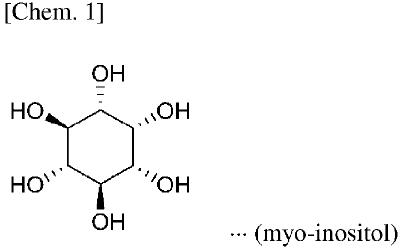

Examples of methods for producing inositol include an extraction method from rice bran, a chemical synthesis method, and a fermentation method.

### · Sugar

A sugar constituting the inositol derivative may be a monosaccharide or an oligosaccharide. Here, the monosaccharide means a sugar that is not further hydrolyzed, and a compound that becomes a constituent element when forming a polysaccharide. The monosaccharide can also be said a minimum unit of sugars. The oligosaccharide is a sugar oligomer consisting of multiple monosaccharides bound by glycosidic bonds. The oligosaccharide may be 2 to 23, 3 to 19, 5 to 10, or 2 to 8 monosaccharides bound to each other.

### ·· Monosaccharide

Specific examples of monosaccharides include glucose, fructose, galactose, ribose, xylose, mannitol, sorbitol, xylitol, erythritol, and pentaerythritol.

### · Oligosaccharide

Specific examples of oligosaccharides include: disaccharides such as sucrose, lactose, maltose, isomaltose, trehalose, cellobiose, and maltitol; trisaccharides such as raffinose, melezitose, and maltotriose; tetrasaccharides such as stachyose; hexasaccharides such as α-cyclodextrin; heptasaccharides such as β-cyclodextrin; and octasaccharides such as γ-cyclodextrin.

Among the above, glucose or an oligosaccharide containing glucose as a monosaccharide unit is preferable as the sugar constituting the inositol derivative.

Here, the monosaccharide unit means a chemical structure corresponding to a monosaccharide, and can also be said to be a chemical structure derived from a monosaccharide.

The above-described oligosaccharide containing glucose as a monosaccharide unit may be an oligosaccharide consisting of only multiple glucoses bound by glycosidic bonds, or may be an oligosaccharide consisting of at least one molecule of glucose and multiple non-glucose sugars bound by glycosidic bonds.

The molecular weight of the oligosaccharide containing glucose as a monosaccharide unit may be, for example, about 300 to 3,000.

In the inositol derivative in the viral infection suppressant of the present embodiment, a sugar may be bound to any one or two or more of six hydroxyl groups present in the inositol molecule. For example, one or multiple monosaccharides may be bound to one molecule of inositol, one or multiple oligosaccharides may be bound to one molecule of inositol, or one or multiple monosaccharides and one or multiple oligosaccharides may be bound to one molecule of inositol.

In the inositol derivative in the present embodiment, the total number of sugars (monosaccharides and/or oligosaccharides) bound to one molecule of inositol is one or more in terms of monosaccharide units, and may be, for example, two or more, three or more, four or more, or ten or more.

Here, the expression "in terms of monosaccharide units" indicates how many monosaccharide units constitute the sugars bound to one molecule of inositol. When multiple sugars are bound to one molecule of inositol, the expression means the sum of the number of monosaccharide units of the multiple sugars.

Specifically, with conversion of a disaccharide to monosaccharide units, it is two, and with conversion of a trisaccharide to monosaccharide units, it is three. In addition, in a case where a disaccharide and a trisaccharide are bound to one molecule of inositol, the number is five with conversion thereof to monosaccharide units.

More specifically, with conversion of a monosaccharide such as glucose, fructose, galactose, ribose, xylose, mannitol, sorbitol, xylitol, erythritol, or pentaerythritol to monosaccharide units, it is one.

In addition, with conversion of a disaccharide such as sucrose, lactose, maltose, isomaltose, trehalose, cellobiose, or maltitol to monosaccharide units, it is two.

In addition, with conversion of a trisaccharide such as raffinose, melezitose, or maltotriose to monosaccharide units, it is three.

In addition, with conversion of a tetrasaccharide such as stachyose to monosaccharide units, it is four, with conversion of a hexasaccharide such as α-cyclodextrin to monosaccharide units, it is six, with conversion of a heptasaccharide such as β-cyclodextrin to monosaccharide units, it is seven; and with conversion of an octasaccharide such as γ-cyclodextrin to monosaccharide units, it is eight.

In the inositol derivative in the present embodiment, β-cyclodextrin is preferably used as a raw material sugar from the viewpoint that a highly purified inositol derivative is likely to be obtained. β-cyclodextrin is industrially inexpensive and can be stably supplied.

In this case, sugars constituting the inositol derivative contain glucose as a constituent unit.

On the other hand, when cheaper starch or the like is used as a raw material sugar for inositol derivatives, various sugars are transferred to various locations during synthesis of inositol derivatives, and therefore, the degree of purification of the resulting inositol derivatives tends to be unstable.

The inositol derivative in the present embodiment may be in the form of a pharmaceutically acceptable salt.

In the present specification, the term "pharmaceutically acceptable salt" means a salt form that does not inhibit physiological activity of the inositol derivative. Pharmaceutically acceptable salts of the inositol derivative are not particularly limited, but examples thereof include salts with alkali metals (such as sodium and potassium); salts with alkaline earth metals (such as magnesium and calcium); salts with organic bases (such as pyridine and triethylamine), and salts with amines.

In addition, the inositol derivative may be in the form of a solvate. Furthermore, the inositol derivative may be in the form of a solvate of a salt of the inositol derivative. Solvates are not particularly limited, and examples thereof include hydrates and ethanol solvates.

In the viral infection suppressant of the present embodiment, the inositol derivatives may be used alone or in combination of two or more thereof.

Among the above, the inositol derivative in the viral infection suppressant of the present embodiment is preferably a mixture of two or more types of inositol derivatives, more preferably a mixture of 2 to 40 types of inositol derivatives, still more preferably a mixture of 2 to 30 types of inositol derivatives, and particularly preferably a mixture of 10 to 30 types of inositol derivatives.

Among the above, the inositol derivatives in the viral infection suppressant of the present embodiment preferably include an inositol derivative in which the total number of sugars bound to one molecule of inositol is 10 or more in terms of monosaccharide units.

In addition, the inositol derivatives in the viral infection suppressant of the present embodiment are preferably inositol derivatives in which glucose or an oligosaccharide containing glucose as a monosaccharide unit is bound to inositol, and are preferably a mixture of two or more types of the inositol derivatives, more preferably a mixture of 2 to 40 types of the inositol derivatives, still more preferably a mixture of 2 to 30 types of inositol derivatives, and particularly preferably a mixture of 10 to 30 types of the inositol derivatives.

The inositol derivatives in the viral infection suppressant of the present embodiment include an inositol derivative in which glucose or an oligosaccharide containing glucose as a monosaccharide unit is bound to inositol, and preferably include an inositol derivative in which the total number of glucose and oligosaccharides containing glucose as a monosaccharide unit bound to one molecule of inositol is 10 or more in terms of monosaccharide units.

The method for producing an inositol derivative is not particularly limited, and inositol derivatives can be appropriately produced through conventionally known methods. For example, inositol may be reacted with a cyclodextrin, which is a type of oligosaccharide, in the presence of cyclodextrin glucanotransferase to synthesize an inositol derivative (for example, refer to Japanese Unexamined Patent Application, First Publication No. S63-196596). Alternatively, glucosyl phosphite may be used as a sugar donor to synthesize an inositol derivative through a method of obtaining a glucosyl product (for example, refer to Japanese Unexamined Patent Application, First Publication No. H6-298783).

The viral infection suppressant of the present embodiment has an effect of suppressing binding of viruses to target cells. The viral infection suppressant of the present embodiment particularly has an effect of suppressing binding of SARS viruses to target cells and has an effect of inhibiting binding of SARS viruses to human ACE2. For this reason, the viral infection suppressant of the present embodiment has an effect of suppressing viruses, especially SARS viruses, from infecting humans.

In addition, the viral infection suppressant of the present embodiment can be applied to the body surface of an animal (such as human skin) or an article to impart antiviral properties to the applied surface. For this reason, viral infection due to contact with the skin or the article can be suppressed by applying the viral infection suppressant of the present embodiment to the body surface of an animal or the surface of an article.

Animals to which the viral infection suppressant of the present embodiment can be applied are not particularly limited, but examples thereof include humans and non-human animals (such as pets such as dogs, cats, rabbits, and hamsters; and livestock such as cows, pigs, horses, sheep, and goats).

Articles to which the viral infection suppressant of the present embodiment can be applied are not particularly limited. Examples of the articles include garments such as clothes; daily necessities such as towels, handkerchiefs, toothbrushes, and tableware items; food packages; industrial instruments such as various machine tools, various tools, and various measurement instruments; medical supplies such as medical gloves, medical clothes, and medical protective equipment; and various medical instruments.

Examples of methods of applying the viral infection suppressant of the present embodiment include: a method of applying the viral infection suppressant of the present embodiment to tissue paper, kitchen paper, non-woven fabrics, and the like through soaking; a method of blending it with a softener or the like for clothes, immersing clothes in the softener or the like, and washing the clothes to make it soak in the clothes; a method of immersing the surface of an article in a solution containing the viral infection suppressant of the present embodiment and then drying it; a method of spraying a spray containing the viral infection suppressant of the present embodiment on the application surface; a method of formulating it into nasal drops to directly treat it to the human body; and a method of preparing it as a spray to treat it in a space.

In a case where the article is a textile product such as a cloth or a towel, the viral infection suppressant of the present embodiment may be blended into a fiber to manufacture a fiber product using the fiber. Alternatively, a fiber product may be immersed in a solution containing the viral infection suppressant of the present embodiment and dried. Alternatively, a solution containing the viral infection suppressant of the present embodiment may be sprayed on a fiber product.

The viral infection suppressant of the present embodiment may be used by being sprayed on the surface of an animal. Animals include humans and non-human animals. In addition, the viral infection suppressant of the present embodiment may be incorporated into nasal drops and applied to the nasal mucosa in the form of the nasal drops.

Examples of methods of using the viral infection suppressant of the present embodiment include a method including applying the viral infection suppressant to the surface of an article. Accordingly, it is possible to impart antiviral properties to the surface of an article and suppress viral infection in humans or animals that have come into contact with the article.

Examples of methods of using the viral infection suppressant of the present embodiment include a method including spraying the viral infection suppressant on the body surfaces of humans or animals. Accordingly, it is possible to impart antiviral properties to the body surfaces of humans or animals and suppress viral infection in humans or animals, or in humans or animals that have come into contact with the humans or animals.

Examples of methods of using the viral infection suppressant of the present embodiment include a method including spraying or blending the viral infection suppressant into a fiber. Accordingly, it is possible to impart antiviral properties to the fiber and suppress viral infection in humans or animals that have come into contact with the fiber.

The present invention also provides use of an inositol derivative to impart antiviral properties to articles, the body surfaces of animals (including humans), or fibers.

Viruses to which the viral infection suppressant of the present embodiment can be applied are not particularly limited, but examples thereof include at least one virus selected from the group consisting of the family *Orthomyxoviridae,* the family *Paramyxoviridae*, the family *Retroviridae*, the family *Filoviridae*, and the family *Coronaviridae.* Among these, it is suitably applicable to viruses of the family *Orthomyxoviridae* or the family *Coronaviridae.* Examples of the family *Orthomyxoviridae* include influenza viruses (the genus *Influenza A virus*, the genus *Influenza B virus*, and the genus *Influenza C virus*), the genus *Isavirus*, the genus *Quaranjavirus*, and the genus *Thogotovirus.* Examples of viruses of the family *Coronaviridae* include the genus *Alphacoronavirus*, the genus *Betacoronavirus*, the genus *Deltacoronavirus*, and the genus *Gammacoronavirus.* Examples of the genus *Betacoronavirus* include the subgenus *Embecovirus*, the subgenus *Hibecovirus*, the subgenus *Merbecovirus*, the subgenus *Nobecovirus*, and the subgenus *Sarbecovirus.* Examples of the subgenus *Sarbecovirus* include SARS viruses (SARS-related coronavirus) such as SARS-CoV and SARS-CoV-2.

### (Composition for suppressing viral infection)

A composition for suppressing viral infection of the present embodiment contains the viral infection suppressant of the above-described embodiment and a pharmaceutically acceptable carrier.

A pharmaceutically acceptable carrier or the like may be appropriately added to the viral infection suppressant of the above-described embodiment to prepare a composition for suppressing viral infection. Examples of viruses to which the composition for suppressing viral infection can be applied are the same as above.

The composition for suppressing viral infection of the present embodiment can be produced through formulation by mixing an inositol derivative, a pharmaceutically acceptable carrier, and other components depending on the circumstances according to a usual method (for example, the method described in the Japanese Pharmacopoeia).

The "pharmaceutically acceptable carrier" in the present specification means a carrier that does not inhibit physiological activity of active components and does not exhibit substantial toxicity to a subject to which it is administered.

The expression "not exhibiting substantial toxicity" means that the component does not exhibit toxicity to an administration subject at a dosage normally used.

The pharmaceutically acceptable carrier is not particularly limited, but examples thereof include excipients, binders, disintegrants, lubricants, stabilizers, diluents, injection solvents, humectants, touch improvers, surfactants, polymer-thickening and gelling agents, solvents, propellants, antioxidants, reducing agents, oxidizers, chelating agents, acid, alkalis, powder, inorganic salts, water, metal-containing compounds, unsaturated monomers, polyhydric alcohols, polymer additives, moisturizers, thickeners, viscosity-imparting substances, oily raw materials, liquid matrices, fat-soluble substances, and polymeric carboxylates.

Such pharmaceutically acceptable carriers may be used alone or in combination of two or more thereof.

In addition, other components are not particularly limited, but examples thereof include preservatives, antibacterial agents, ultraviolet absorbers, whitening agents, vitamins and their derivatives, antiphlogistic agents, anti-inflammatory agents, hair growth agents, blood circulation promoters, stimulants, hormones, anti-wrinkle agents, anti-aging agents, tightening agents, cool-feeling agents, warm-feeing agents, wound-healing promoters, irritation relievers, analgesics, cell activators, plant/animal/microorganism extracts, seed oils, antipruritic agents, keratin-peeling and dissolving agents, antiperspirants, refreshing agents, astringent agents, enzymes, nucleic acids, fragrances, pigments, Colorants, dyes, pigments, anti-inflammatory analgesics, antifungal agents, antihistamines, hypnotic and sedative agents, tranquilizers, antihypertensive agents, hypotensive diuretics, antibiotics, anesthetics, antibacterial substances, coronary vessel dilators, herbal medicines, antipruritic agents, keratin-softening and peeling agents, ultraviolet-blocking agents, bactericidal agents, antioxidant substances, pH adjusters, additives, and metal soap. Furthermore, specific examples of plant/animal/microorganism extracts include *Lapsana communis* flowers/leaves/stems and tea leaves. Specific examples of seed oils include *Moringa oleifera* seed oil. Specific examples of fragrances include perillaldehyde.

Other components may be used alone or in combination of two or more thereof.

The total content of inositol derivatives in the composition for suppressing viral infection of the present embodiment based on the total mass of the composition for suppressing viral infection may be, for example, 0.1 to 5.0 mass%, 0.2 to 3.0 mass%, or 0.5 to 2.0 mass%.

The total content of inositol derivatives means the content of one type of inositol derivative when the compound is used alone and means the content of two or more types of inositol derivatives when these compounds are incorporated.

Specific examples of the composition for suppressing viral infection of the present embodiment include a spray composition, an aerosol composition, a skin spray composition, and a nasal drop composition.

### «Spray composition»

A spray composition is a composition used for spraying. The spray composition is prepared as a spray liquid for spraying to be filled into a spray container, and then used by being filled into a spray container. The spray liquid for spraying may contain ethanol, an antibacterial agent, and the like in addition to inositol derivatives. The spray composition is applied to the surface of an article through, for example, spraying. Inositol derivatives contained in a spray composition impart antiviral properties to the surface of an article to which the spray composition is applied, thereby reducing the risk of viral infection due to contact with the surface of the article. Examples of applicable articles include the same ones exemplified for the viral infection suppressant.

### «Aerosol composition»

An aerosol composition is a composition used for aerosol spraying. The aerosol composition is prepared as an aerosol spray liquid to be filled into an aerosol container, and then used by being filled into an aerosol container. The aerosol spray liquid may contain an anionic sodium salt additive, titanium oxide, and the like in addition to inositol derivatives. The aerosol composition is applied to the surface of an article through, for example, aerosol spraying. Inositol derivatives contained in an aerosol composition impart antiviral properties to the surface of an article to which the aerosol composition is applied, thereby reducing the risk of viral infection due to contact with the surface of the article. Examples of applicable articles include the same ones exemplified for the viral infection suppressant.

### <<Skin spray composition>>

A skin spray composition is a composition used for spraying on human skin. The skin spray composition is prepared as a skin spray liquid to be filled into a spray container, and then used by being filled into a spray container. The skin spray liquid may contain titanium oxide, ethanol, sodium hyaluronate, and the like in addition to inositol derivatives. The skin spray composition is applied to human skin through, for example, spraying. Inositol derivatives contained in a skin spray composition impart antiviral properties to the surface of the skin to which the skin spray composition is applied, thereby reducing the risk of viral infection due to contact with the skin. Applicable sites are not particularly limited, but examples thereof include hands, arms, face, neck, feet, legs, and torso.

### «Nasal drop composition»

A nasal drop composition is a composition used as nasal drops. The nasal drop composition is prepared as a nasal drop solution to be filled into a nasal drop container, and then used by being filled into a nasal drop container. The nasal drop solution may contain an antiallergic agent, an antibacterial agent, and the like in addition to inositol derivatives. The nasal drop composition is applied to the nasal mucosa as nasal drops. Inositol derivatives contained in a nasal drop composition inhibit binding of viruses to target cells, thereby suppressing viral infection via the nasal mucosa.

### <<Composition for medical supplies>>

A composition for medical supplies is a composition used by being applied to the surface of medical supplies. The composition for medical supplies is used by being applied to medical supplies. Inositol derivatives contained in a composition for medical supplies impart antiviral properties to the surface of medical supplies to which the composition for medical supplies is applied, thereby reducing the risk of viral infection due to contact with the medical supplies. Examples of medical supplies include medical gloves, medical clothes, and medical protective equipment. When a composition for medical supplies is used in a medical glove, the glove may be immersed in the composition for medical supplies, and then dried. The inositol derivatives remain on the surface of the dried glove, thereby exhibiting antiviral properties.

### <<Pharmaceutical Composition>>

The composition for suppressing viral infection of the present embodiment may be a pharmaceutical composition for preventing viral infection or viral infectious diseases.

In the pharmaceutical composition, carriers commonly used for pharmaceuticals can be used as pharmaceutically acceptable carriers in addition to those listed above. For example, general raw materials described in the Japanese Pharmacopoeia, non-official Pharmaceutical Standards, Japanese Pharmaceutical Excipients 2013 (Yakuji Nippo, Limited, 2013), the Japanese Pharmaceutical Excipients Dictionary 2016 (edited by International Pharmaceutical Excipients Council Japan, Yakuji Nippo, Limited, 2016), and Handbook of Pharmaceutical Excipients, 7th edition (Pharmaceutical Press, 2012) can be used. In the pharmaceutical composition, such pharmaceutically acceptable carriers may be used alone or in combination of two or more thereof.

The pharmaceutical composition may contain other components in addition to the viral infection suppressant and the pharmaceutically acceptable carriers. Other components are not particularly limited, and general pharmaceutical excipients can be used. In addition, as other components, active ingredients other than the viral infection suppressant can also be used. As the pharmaceutical excipients and active ingredients as other components, for example, general raw materials described in the Japanese Pharmacopoeia, non-official Pharmaceutical Standards, Japanese Pharmaceutical Excipients 2013 (Yakuji Nippo, Limited, 2013), the Japanese Pharmaceutical Excipients Dictionary 2016 (edited by International Pharmaceutical Excipients Council Japan, Yakuji Nippo, Limited, 2016), and Handbook of Pharmaceutical Excipients, 7th edition (Pharmaceutical Press, 2012) can be used in addition to those listed above. In the pharmaceutical composition, other components may be used alone or in combination of two or more thereof.

The dosage form of the pharmaceutical composition is not particularly limited, and may be any dosage form commonly used as a pharmaceutical preparation. Examples thereof include orally administered dosage forms such as tablets, coated tablets, pills, powders, granules, capsules, liquid agents, suspensions, and emulsions; and parenterally administered dosage forms such as injections, suppositories, mouthwash, eye drops, topical skin applications, and nasal drops. Pharmaceutical compositions in these dosage forms can be formulated according to standard methods (for example, methods described in the Japanese Pharmacopoeia). As the pharmaceutical compositions, parenteral preparations are preferable, and topical skin applications or nasal drops are more preferable.

A method for administering a pharmaceutical composition is not particularly limited, and administration can be performed through methods commonly used as methods for administering a pharmaceutical composition. For example, a pharmaceutical composition may be administered orally, administered intravenously, intraarterially, intramuscularly, intradermally, subcutaneously, intraperitoneally as an injection, an infusion preparation, or the like, administered intrarectally as suppositories, administered to the eyes as eye drops, used for washing the mouth as mouthwash, administered to the skin as a topical skin application, or administered to the nose as nasal drops.

The dosage of the pharmaceutical composition can be a therapeutically effective amount. The therapeutically effective amount may be appropriately determined depending on the patient's symptoms, body weight, age, sex, and the like, the dosage form of the pharmaceutical composition, the administration method, and the like.

For example, in a case of a topical skin application, examples of the dosage of the pharmaceutical composition per skin area to be applied as the total content of inositol derivatives include 0.01 to 100 µg/cm², 0.1 to 50 µg/cm², or 1 to 20 µg/cm². For example, in a case of nasal drops, examples of the dosage of the pharmaceutical composition per nasal mucosa area to be applied as the total content of inositol derivatives include 0.01 to 100 µg/cm², 0.1 to 50 µg/cm², or 1 to 20 µg/cm². The pharmaceutical composition may contain a therapeutically effective amount of inositol derivatives. The therapeutically effective amount of inositol derivatives in the pharmaceutical composition may be an amount of inositol derivatives effective for preventing viral infectious diseases. The therapeutically effective amount of inositol derivatives may be, for example, an amount capable of inhibiting binding of viruses to target cells, an amount capable of inhibiting binding of SARS viruses to human ACE2, an amount capable of reducing viral activity, or an amount capable of suppressing viral infection.

The administration interval of the pharmaceutical composition is not particularly limited, and may be appropriately determined through the administration method and the like. For example, it can be set to once a day or about two to three times a day and the like.

Since the pharmaceutical composition of the present embodiment contains inositol derivatives as an active component, it is effective for preventing viral infection or viral infectious diseases. For example, by applying the pharmaceutical composition of the present embodiment to the skin such as hands or face as a topical skin application, it is possible to reduce viral activity at the application site and suppress viral infection through the application site.

By applying the pharmaceutical composition of the present embodiment to the nasal mucosa as nasal drops, it is possible to suppress viral infection through the nasal mucosa.

Accordingly, the pharmaceutical composition of the present embodiment is effective for preventing viral infection.

### (Other embodiments)

In one embodiment, the present invention provides a method for preventing viral infection, the method including a step of administering an inositol derivative to a subject. In addition, the present invention provides a method for preventing viral infection or a viral infectious disease, the method including a step of applying an inositol derivative to the skin of a subject. The present invention provides a method for preventing viral infection or a viral infectious disease, the method including a step of nasally administering an inositol derivative to a subject.

In one embodiment, the present invention provides a method for imparting antiviral properties to the surface of an article, the method including a step of applying an inositol derivative to the surface of the article. In addition, the present invention provides a method for imparting antiviral properties to the surface of an article, the method including a step of spraying or aerosol spraying an inositol derivative on the surface of the article. In addition, the present invention provides a method for producing an antiviral article, the method including a step of adding an inositol derivative to a raw material for an article and a step of producing the article from the raw material. In addition, the present invention provides a method for imparting antiviral properties to the surface of an article, the method including a step of immersing an article in an aqueous solution containing an inositol derivative and a step of drying the article after immersion.

In one embodiment, the present invention provides an inositol derivative for suppressing viral infection. In addition, the present invention provides an inositol derivative for preventing viral infection or a viral infectious disease.

In one embodiment, the present invention provides use of an inositol derivative for producing a composition for suppressing viral infection. In addition, the present invention provides use of an inositol derivative for producing a pharmaceutical composition for preventing viral infection. In addition, the present invention provides use of an inositol derivative for producing a pharmaceutical composition for preventing a viral infectious disease.

In one embodiment, the present invention provides use of an inositol derivative for suppressing viral infection. In addition, the present invention provides use of an inositol derivative for preventing viral infection. In addition, the present invention provides use of an inositol derivative for preventing a viral infectious disease.

### [Examples]

Hereinafter, the present invention will be described in more detail using examples and comparative examples, but is not limited to the following examples.

### [Inositol derivative]

In the following examples and formulation examples, an inositol derivative A produced through a method disclosed in PCT International Publication No. WO2019/045113 was used.

Specifically, myo-inositol (manufactured by Tsuno Rice Fine Chemicals) was reacted with β-cyclodextrin (manufactured by Ensuiko Sugar Refining Co., Ltd.) in the presence of cyclodextrin glucanotransferase (manufactured by Novozymes A/S) to produce an inositol derivative which is a mixture of inositol derivatives in which glucose or an oligosaccharide containing glucose as a monosaccharide unit is bound to myo-inositol.

The obtained inositol derivative was analyzed through liquid chromatography mass-spectrometry (LC-MS). The results show that the proportion of molecules with one glucose in glucose chains bound to myo-inositol was 12 mass%, the proportion of molecules with two glucoses therein was 30 mass%, the proportion of molecules with three glucoses therein was 9 mass%, the proportion of molecules with four glucoses therein was 12 mass%, and the proportion of molecules with five glucoses therein was 2 mass%.

### (Example 1)

### [Inhibitory effect on binding of SARS-CoV-2 to membrane proteins]

A COVID-19 Spike-ACE2 Binding Assay Kit manufactured by RayBiotech Life, Inc. was used to investigate an inhibitory effect of the inositol derivative A on the binding of the SARS-CoV-2 virus on human ACE2.

The inositol derivative A was dissolved in purified water to prepare 0.5% (V/V), 1% (V/V), and 2 (V/V)% inositol derivative A aqueous solutions. In addition, myo-inositol was dissolved in purified water to prepare a 2% (V/V) myo-inositol aqueous solution. According to the instructions included in the kit, the inositol derivative A aqueous solutions or the myo-inositol aqueous solution were each mixed with an ACE2 protein solution included in the kit. The mixed solution was added to a COVID-19 S-protein adsorption plate included in the kit to cause a reaction under shaking at 4°C overnight. A control was prepared by adding the same amount of water instead of the aqueous solutions. Thereafter, a horseradish-derived peroxidase-conjugated anti-goat IgG antibody, which is a secondary antibody included in the kit, was added to the plate to cause a reaction. After washing the plate, peroxidase chromogenic substrate 3,3',5,5'-tetramethylbenzimide (included in the kit) was added to the plate to cause a chromogenic reaction. After the chromogenic reaction, the absorbance was measured at a wavelength of 450 nm using a microplate reader (manufactured by Tecan Life Sciences).

The results are shown in Table 1. The virus binding rate in each example is shown as a relative binding rate when the virus binding rate of the control was set to 1.00. It was confirmed from the results shown in Table 1 that the inositol derivative A had an effect of inhibiting the binding of SARS-CoV-2 to ACE2. On the other hand, no binding inhibitory effect was observed in myo-inositol. These results indicate that inositol derivatives have an inhibitory effect on the binding of SARS-CoV-2 to membrane proteins.

**[Table 1]**

| Binding inhibitory effect on binding of SARS-CoV-2 to ACE2 | | |
|---|---|---|
| | Concentration (% (V/V)) | Virus binding rate |
| Control | 0 | 1.00 |
| Inositol derivative A | 0.01 | 0.95 |
| | 0.1 | 0.87 |
| | 1 | 0.77 |
| | 2 | 0.60 |
| Myo-inositol | 2 | 1.00 |

### (Example 2)

### [Example of application to medical supplies]

A commercially available natural rubber disposable glove (Ansell, TouchNTuff 69-318) was immersed in a 1% (V/V) inositol derivative A aqueous solution or myo-inositol aqueous solution for 10 minutes. Water was drained from the glove after immersion, and the glove was dried in an oven at about 60°C for 3 hours. A 5 cm × 5 cm square film was cut from the dried glove, and the antiviral properties against an influenza virus (influenza A/Kitakyushu/159/93 (H3N2)) were measured through a plaque method.

The cut-out sample was placed in a plastic Petri dish, 50 µL of an influenza virus suspension was added dropwise thereto, and the mixture was allowed to act at room temperature for 1 hour. Thereafter, 950 µL was added to SCDLP medium, and the virus was washed and collected through pipetting. Thereafter, each virus washout solution was serially diluted 10-fold with MEM diluent until it became one hundredth to one millionth. 100 µL of the sample solution was inoculated into Madin-Darby Canine Kidney (MDCK: derived from canine kidney tubular epithelial cells) cells cultured in a Petri dish. After standing for 1 hour to allow the virus to adsorb to the cells, 0.7% agar medium was overlaid and cultured in a 5% CO₂ incubator for 48 hours at 34°C. Thereafter, formalin fixation was performed, and formed plaques were stained through methylene blue staining, and the number of plaques was counted. The viral infectivity titer (PFU/0.1 mL, Log10); (PFU: plaque-forming units) was calculated based on the number of plaques counted. As a control, a virus solution that had not come into contact with the sample was used instead of the washout solution from the sample cut out from the glove.

The results are shown in Table 2. The viral infectivity titer in a case where the virus came into contact with the inositol derivative A-applied sample was lower than that in a case where the virus came into contact with the control- and myo-inositol-applied samples. It was possible to confirm from these results that applying inositol derivatives to medical supplies exhibits a high antiviral effect against influenza viruses.

**[Table 2]**

| Evaluation of antiviral properties against influenza virus | |
|---|---|
| | Viral infectivity titer (PFU/0.1 mL, Log10) |
| Control | 6.78 |
| Inositol derivative A | 2.8 |
| Myo-inositol | 4.1 |

### (Example 3)

### [Example of application to non-woven fabric]

A non-woven fabric made of polyethylene terephthalate with a basis weight of 80 g/m² was immersed in a 2% (V/V) inositol derivative A aqueous solution or myo-inositol aqueous solution so that the pickup rate was 200%. Thereafter, the non-woven fabric was dried at 110°C to prepare a processed non-woven fabric. The pickup rate was calculated by the following equation. Pickup rate + (weight of non-woven fabric after immersion - weight of non- woven fabric before immersion) / weight of non-woven fabric before immersion

The processed non-woven fabric prepared as described above was subjected to an antiviral test against a coronavirus (SARS-CoV-2) according to ISO 18184. The test method was almost the same as that in Example 2. As a control, a virus solution that had not come into contact with the processed non-woven fabric was used instead of the washout solution from the processed non-woven fabric. The viral infectivity titer (PFU/0.1 mL, Log10) was calculated based on the number of plaques formed.

The results are shown in Table 3. In a case were a processed non-woven fabric coated with an inositol derivative A was used, the viral infectivity titer of SARS-CoV-2 was below the detection limit value, exhibiting high antiviral properties. It was possible to confirm from these results that applying an inositol derivative to a non-woven fabric exhibited a high antiviral effect against a coronavirus.

**[Table 3]**

| Evaluation of antiviral properties against coronavirus | |
|---|---|
| | Viral infectivity titer (PFU/0.1 mL, Log10) |
| Control | 6.63 |
| Inositol derivative A | < 1.3 |
| Myo-inositol | 2.7 |

### (Example 4)

A spray liquid for spraying of Formulation Example 1 shown in Table 4 was filled into a spray container. The spray liquid was sprayed from this spray container on the evaluation surface. Inositol derivatives were detected in an amount of 0.1 to 10 µg/cm² on the evaluation surface on which the spray liquid was sprayed. This indicated that a sufficient effect of suppressing viral infection can be expected by spraying the spray liquid for spraying which contains 2 mass% inositol derivatives.

**[Table 4]**

| Formulation Example 1: Spray liquid for spraying | |
|---|---|
| Component | Formulation (mass%) |
| Inositol derivative A | 2 |
| Carboxyvinyl polymer | 1 |
| Ethyl alcohol | 30 |
| Phenoxyethanol | 0.2 |
| Water | 66.3 |

### (Example 5)

An aerosol spray liquid of Formulation Example 2 shown in Table 5 was filled into a spray container. The spray liquid was sprayed from this aerosol spray container on the evaluation surface. Inositol derivatives were detected in an amount of 0.5 to 10 µg/cm² on the evaluation surface on which the spray liquid was sprayed. This indicated that a sufficient effect of suppressing viral infection can be expected by spraying the aerosol spray liquid containing 2 mass% inositol derivatives.

**[Table 5]**

| Formulation Example 2: Aerosol spray liquid | |
|---|---|
| Component | Formulation (mass%) |
| Inositol derivative A | 2 |
| Ethyl alcohol | 35 |
| Water | 10 |
| Nitrogen gas (propellant) | 54 |

### (Example 6)

A skin spray liquid of Formulation Example 3 shown in Table 6 was filled into a spray container. The spray liquid was sprayed from this spray container on the evaluation surface. Inositol derivatives were detected in an amount of 1 to 10 µg/cm² on the evaluation surface on which the spray liquid was sprayed. This indicated that a sufficient effect of suppressing viral infection can be expected by spraying the skin spray containing 1 mass% inositol derivatives.

**[Table 6]**

| Formulation Example 3: Skin spray liquid | |
|---|---|
| Component | Formulation (mass%) |
| Inositol derivative A | 1 |
| Carboxyvinyl polymer | 1 |
| Ethyl alcohol | 25 |
| Titanium oxide | 0.5 |
| Sodium hyaluronate | 0.5 |
| Phenoxyethanol | 0.2 |
| Water | 71.8 |

### (Example 7)

A nasal drop solution of Formulation 4 shown in Table 7 was filled into a nasal drop container. Inositol derivatives were detected in an amount of 0.1 to 5 µg/cm² on the application surface after the nasal drops were applied thereto. This indicated that a sufficient effect of suppressing viral infection can be expected by applying the nasal drop solution containing 0.5 mass% inositol derivatives.

**[Table 7]**

| Formulation Example 4: Nasal drop solution | |
|---|---|
| Component | Formulation (mass%) |
| Inositol derivative A | 0.5 |
| Carboxyvinyl polymer | 0.5 |
| Sodium chloride | 1 |
| Benzalkonium chloride | 0.2 |
| Ethanol | 0.1 |
| Edetic acid | 0.2 |
| Menthol | Trace amount |
| Surfactant | 0.2 |
| Water | 96.8 |

Preferred examples of the present invention have described above, but the present invention is not limited to the examples. Addition, omission, replacement, and other modifications of the configurations can be made within the scope not departing from the gist of the present invention. The present invention is not limited by the above-described description, but is limited only by the scope of the accompanied claims.

## Claims

1. A viral infection suppressant containing, as an active component, an inositol derivative in which a sugar is bound to inositol.

2. The viral infection suppressant according to claim 1,
wherein the sugar is glucose or an oligosaccharide containing glucose as a constituent unit.

3. The viral infection suppressant according to claim 1,
wherein the inositol is myo-inositol.

4. The viral infection suppressant according to any one of claims 1 to 3,
wherein the virus is at least one virus selected from the group consisting of the family *Orthomyxoviridae*, the family *Paramyxoviridae*, the family *Retroviridae*, the family *Filoviridae*, and the family *Coronaviridae.*

5. The viral infection suppressant according to claim 4,
wherein the virus is SARS-related coronavirus.

6. A method of using a viral infection suppressant, the method comprising:
applying the viral infection suppressant according to any one of claims 1 to 3 to a surface of an article.

7. A method of using a viral infection suppressant, the method comprising:
spraying the viral infection suppressant according to any one of claims 1 to 3 on a body surface of an animal.

8. A method of using a viral infection suppressant, the method comprising:
spraying or blending the viral infection suppressant according to any one of claims 1 to 3 into a fiber.

9. A composition for suppressing viral infection, comprising:
the viral infection suppressant according to any one of claims 1 to 3; and
a pharmaceutically acceptable carrier.

10. The composition for suppressing viral infection according to claim 9,
wherein a total content of the inositol derivative is 0.1 to 5.0 mass%.
